# FASCICULE DE BREVET EUROPEEN

(11) **EP 0 547 988 B1**
(45) Date de publication et mention de la délivrance du brevet: **03.06.1998**
(21) Numéro de dépôt: 92450013.5
(22) Date de dépôt: 06.10.1992
(51) Int. Cl.: A61N 1/05

(54) **Sonde de stimulation, notamment cardiaque, à pôle auxiliaire de connexion électrique**
Reizungssonde, insbesonders herzschrittmachende, versehen mit einem zusätzlichen Pol als elektrische Schaltverbindung
Paving lead, especially cardiac, with an auxiliary pole as electrical connexion

(30) Priorité: 17.12.1991 FR 9115963
(43) Date de publication de la demande: 23.06.1993
(73) Titulaire: Bemurat, Marc, F-33850 Leognan (FR)
(72) Inventeur: Bemurat, Marc, F-33850 Leognan (FR)
(74) Mandataire: Thébault, Jean-Louis

(56) Documents cités:
- EP-A- 0 396 835
- FR-A- 2 654 939
- US-A- 4 154 247
- US-A- 4 499 907

## Description

La présente invention se rapporte en particulier, bien que non exclusivement, aux sondes de stimulation de stimulateur cardiaque.

Actuellement, sur une sonde de stimulation, seule la tête de la sonde peut recevoir une stimulation électrique. Cette tête de sonde étant introduite dans le boîtier de stimulateur cardiaque n'est accessible que lorsque la sonde est déconnectée et sortie du boîtier.

Lors du remplacement d'un boîtier de stimulateur cardiaque, la sonde de stimulation est pratiquement toujours conservée. La manoeuvre consiste à déconnecter la sonde du boîtier, à la sortir de celui-ci, à la réintroduire dans le nouveau boîtier et enfin, à réenfouir sous la peau l'ensemble sonde et boîtier.

Cette manipulation peut prendre plusieurs dizaines de secondes.

Or, dès que la sonde est déconnectée, le patient n'est plus stimulé. Cela n'a pas d'inconvénient si le patient garde un rythme cardiaque propre, mais certains patients dits "dépendants" n'ont aucun rythme spontané. Ils sont donc en arrêt cardiaque jusqu'au moment où la sonde de stimulation est connectée au nouveau boîtier.

Chez un patient dépendant, cette manoeuvre est délicate, voire dangereuse.

Par le document FR-2.654.939 déposé au nom du Demandeur, on connaît déjà un type de sonde de stimulateur cardiaque à pôle de stimulation auxiliaire dans lequel est ménagé, dans la gaine du cordon reliant le boîtier du stimulateur cardiaque au dispositif à électrode de stimulation, une solution de continuité rendant accessible de l'extérieur le ou les conducteurs de la sonde, la solution de continuité étant susceptible d'être isolée de l'extérieur par un moyen mobile tel qu'un manchon monté coulissant sur ledit cordon.

Malgré les avantages au plan pratique et efficacité d'un tel dispositif, la prévision d'un manchon de recouvrement mobile destiné à masquer la solution de continuité dans la gaine isolante et protectrice du ou des conducteurs internes, peut éventuellement poser des problèmes d'étanchéité et donc d'isolement électrique du ou des conducteurs vis à vis du milieu environnant la sonde, qui est conducteur et qui pourrait établir des courts-circuits entre le(s)dit(s) conducteur(s) et le boîtier du stimulateur cardiaque.

Le but de la présente invention, dans son application aux sondes de stimulateur cardiaque, est précisément d'éliminer un tel risque, si minime et théorique soit-il, en proposant de nouveaux agencements aptes à assurer une étanchéité la plus parfaite possible.

A cet effet, la présente invention a pour objet une sonde de stimulation, notamment cardiaque, à pôle auxiliaire de connexion électrique, du type comprenant un cordon souple constitué d'au moins un conducteur électrique spiralé et entouré d'une gaine en matériau isolant et relié, à une extrémité de la sonde, à une tête amovible de connexion au boîtier du stimulateur cardiaque et, à l'autre extrémité, à un dispositif à électrode de stimulation, ladite sonde comportant une structure d'accès étanche audit conducteur électrique de manière à permettre à un moyen de connexion électrique auxiliaire d'assurer la liaison électrique entre ledit conducteur et une source externe de stimulation, caractérisée en ce que ledit conducteur électrique forme un coude au niveau duquel est agencée ladite structure d'accès étanche, celle-ci étant constituée par une pièce en matériau conducteur de l'électricité, disposée en contact direct avec ledit conducteur en formant une dérivation dans l'axe de la partie dudit conducteur tournée vers l'électrode de stimulation, recouverte d'une structure de recouvrement étanche susceptible, à l'extrémité libre de ladite pièce, d'être traversée par ledit moyen de connexion électrique auxiliaire, ce dernier venant alors en contact électrique avec ladite pièce.

Le moyen de connexion électrique auxiliaire peut être une tige d'un tournevis, mais également le mandrin métallique d'insertion de la sonde dans le coeur.

Selon un autre mode de réalisation la présente invention a pour objet une sonde de stimulation, notamment cardiaque, à pôle auxiliaire de connexion électrique, du type comprenant un cordon souple constitué d'au moins un conducteur électrique spiralé et entouré d'une gaine en matériau isolant et relié, à une extrémité de la sonde, à une tête amovible de connexion au boîtier du stimulateur cardiaque et, à l'autre extrémité, à un dispositif à électrode de stimulation, ladite sonde comportant une structure d'accès étanche audit conducteur électrique de manière à permettre à un moyen de connexion électrique auxiliaire d'assurer la liaison électrique entre ledit conducteur et une source externe de stimulation, caractérisée en ce que ledit conducteur électrique comporte, au droit de la structure d'accès étanche, un manchon en matériau conducteur de l'électricité entourant ledit conducteur, ledit manchon étant recouvert par la gaine avec interposition d'une pièce en matériau rigide non conducteur de l'électricité, ladite structure d'accès étant constituée par une vis en matériau conducteur de l'électricité dont la tête est recouverte d'une pastille en matériau isolant, ladite vis étant engagée dans un puits ménagé dans la gaine et dans ladite pièce, de manière à venir par vissage en contact avec ledit manchon, sans rupture de l'étanchéité entre l'intérieur et l'extérieur de la gaine, ladite pastille isolante étant traversable de manière étanche par ledit moyen, permettant de la sorte à ce dernier de venir en contact électrique avec le conducteur, via la vis et le manchon.

Selon encore un autre mode de réalisation, la présente invention concerne une sonde de stimulation, notamment cardiaque, à pôle auxiliaire de connexion électrique, du type comprenant un cordon souple constitué d'au moins un conducteur électrique spiralé et entouré d'une gaine en matériau isolant et relié, à une extrémité de la sonde, à une tête amovible de connexion au boîtier du stimulateur cardiaque et, à l'autre extrémité, à un dispositif à électrode de stimulation, ladite sonde comportant une structure d'accès étanche audit conducteur électrique de manière à permettre à un moyen de connexion électrique auxiliaire d'assurer la liaison électrique entre ledit conducteur et une source externe de stimulation, caractérisée en ce que ledit conducteur électrique comporte, au droit de la structure d'accès étanche, un manchon en matériau conducteur de l'électricité entourant ledit conducteur, ladite gaine étant séparée en deux tronçons, le premier venant en butée contre ledit manchon et le second recouvrant ledit manchon ainsi que l'extrémité du premier troncon, ledit second tronçon assurant l'étanchéité par constriction, ledit moyen étant susceptible d'être inséré sous le second tronçon jusqu'à venir en contact avec le manchon.

Dans la présente description, on entend par cordon la liaison entre le dispositif à électrode(s) de stimulation et le boîtier du stimulateur cardiaque ou analogue, que cette liaison soit constituée d'un cordon unique ou d'un cordon relié par tous moyens d'interconnexion à un raccord dit d'adaptation pourvu d'une tête appropriée, le dispositif de l'invention étant aménagé sur le cordon unique ou sur ledit raccord.

Par ailleurs, l'invention s'applique aussi bien à des sondes unipolaires qu'à des sondes bipolaires à deux conducteurs électriques spiralés, côte-à-côte ou coaxiaux, de même diamètre (sonde bipolaire multifilaire) ou de diamètres différents, l'un des conducteurs spiralés étant logé à l'intérieur de l'autre conducteur spiralé.

L'invention s'applique également à des sondes de défibrillateurs implantables, en offrant, par un tel pôle auxiliaire de connexion électrique, la possibilité de déclencher une arythmie ventriculaire par stimulation de l'électrode distale. Elle s'applique aussi aux stimulateurs anti-tachycardie et, d'une manière générale, à toute sonde implantable, cardiaque ou non, mono ou multiconducteurs, en permettant, avec une telle sonde, soit d'appliquer des stimuli, ou un potentiel électrique, soit de recueillir des signaux électriques en provenance de la sonde, que le boîtier de la sonde soit ou non déconnecté de cette dernière.

D'autres caractéristiques et avantages ressortiront de la description qui va suivre de modes de réalisation du dispositif de l'invention, description donnée à titre d'exemple uniquement et en regard des dessins annexés sur lesquels :
- Figure 1 est une vue en coupe longitudinale axiale d'une sonde de l'invention suivant un premier mode de réalisation ;
- Figure 2 est une vue en coupe transversale suivant la ligne IV-IV de la sonde de la figure 1 ;
- Figure 3 est une vue partielle de la sonde de la figure 1 illustrant une position de non-contact de la vis de liaison électrique ;
- Figure 4 est une vue en coupe longitudinale axiale d'une sonde de l'invention selon un deuxième mode de réalisation ;
- Figure 5 est une vue schématique d'un troisième mode de réalisation ;
- Figure 6 est une vue schématique d'un quatrième mode de réalisation, et
- Figure 7 est une vue schématique d'un cinquième mode de réalisation.

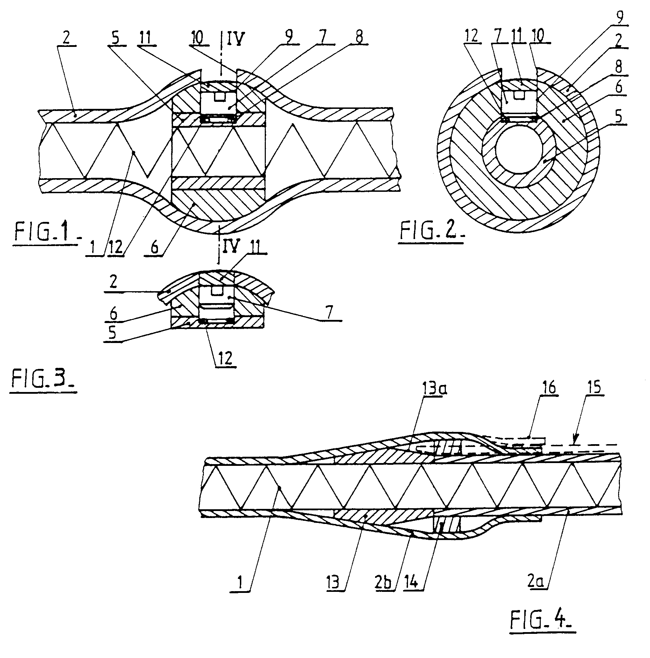

Dans le mode de réalisation représenté par les figures 1 à 3, le conducteur spiralé interne 1 est soudé ou relié à un manchon externe en matériau conducteur de l'électricité 5, lui-même entouré d'un manchon 6 en matériau rigide isolant électrique, par exemple une matière plastique. Le manchon 6 est recouvert par la gaine 2, par exemple en silicone, de la sonde.

Le contact électrique entre le manchon 5 et la source externe de stimulation est opéré par l'intermédiaire d'une vis métallique 7 logée dans un puits taraudé ménagé radialement à la sonde. Ce puits comporte une partie 8 ménagée dans la face externe du manchon 5 et qui ne traverse pas ce dernier, une partie 9 ménagée au travers de la paroi du manchon isolant 6 et une partie 10 traversant la gaine 2.

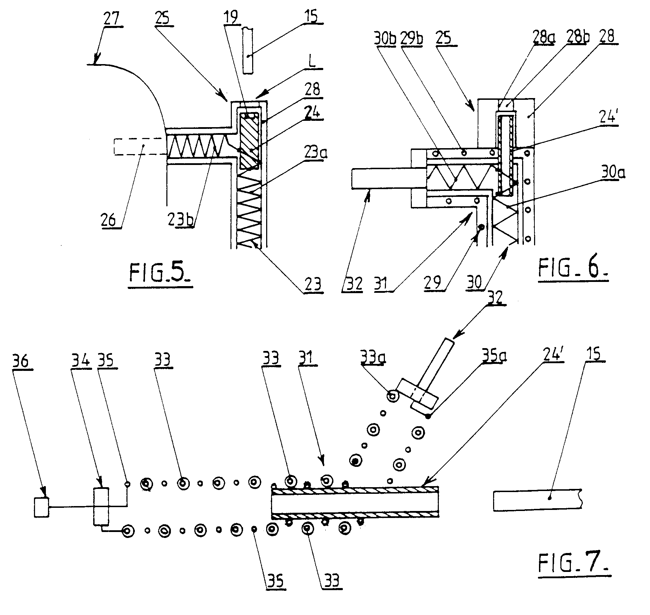

La tête de la vis 7 est, dans le mode de réalisation représenté, recouverte d'une pastille cylindrique 11 en silicone ou analogue, "éventuellement munie d'un passage de part en part, normalement obturé de manière étanche du fait de l'élasticité du matériau. Un tel agencement est bien connu et utilisé pour recouvrir les vis de connexion de la sonde à un boîtier de stimulation cardiaque et permet d'accéder à ces vis par un tournevis sans nuire à l'étanchéité de l'enveloppe". La pastille 11 obture la partie de puits 10 lorsque la vis 7 est (figure 5) suffisamment éloignée du manchon métallique 5 pour ne plus être en contact avec lui.

Dans le fond du puits (8) est disposée une rondelle annulaire 12 formant butée pour la vis 7 en sorte de ménager entre cette dernière et le manchon 5, lorsque la vis (figure 2) est en position de vissage à fond établissant le contact entre la vis 7 et le manchon 5, une chambre de compression de l'air emprisonné.

L'engagement au travers de la pastille 11 d'une tige de tourne-vis permet de visser la vis 7 pour établir (figure 2), via le manchon 5, la vis 7 et le tourne-vis, une liaison électrique entre le conducteur 1 et une source auxiliaire de stimulation, ou au contraire, isoler électriquement, et, ce, de manière totalement étanche, la vis 7 du manchon 5.

Il est à noter que la pastille 11 peut éventuellement être supprimée, la tête de la vis 7 étant alors à nu.

Le mode de réalisation de la figure 4 comporte une autre structure en matériau élastique traversable de manière étanche et propre à assurer une liaison électrique temporaire entre le conducteur spiralé interne de la sonde et l'extérieur.

Dans ce mode de réalisation de la figure 4, le conducteur spiralé interne 1 est soudé à un manchon externe 13 en matériau conducteur de l'électricité. La gaine est séparée en deux tronçons, l'un (2a) venant en butée contre le manchon 13, et, l'autre (2b) recouvrant le manchon 13 et l'extrémité du tronçon 2a.

Ladite structure traversable est constituée par un élément annulaire 14 en un matériau élastique tel que le silicone, interposé entre les parties en recouvrement des deux tronçons 2a,2b. L'élément 14 est formé par exemple d'une bague munie d'un ou plusieurs passages parallèles à l'axe du conducteur 1, pré-formés, du type indiqué plus haut.

L'élément 14 peut être formé de tronçons disposés côte à côte annulairement, chaque tronçon étant muni d'un passage pré-formé parallèle à l'axe du conducteur.

La partie du tronçon 2b recouvrant la bague 14 se plaque contre cette dernière ainsi que contre le tronçon 2a par effet d'élasticité. Pour une meilleure étanchéité, la bague 14 est soudée aux parties de gaine 2a, 2b.

La face externe du manchon 13 est avantageusement biconique afin de constituer une surface inclinée 13a assurant le contact avec l'extrémité d'une tige métallique 15, par exemple d'un tourne-vis, engagée sous l'extrémité libre 16 du tronçon 2b, en sorte de traverser la bague 14 jusqu'à toucher ladite surface 13a.

La tige 15 permet ainsi d'établir une liaison électrique momentanée entre le conducteur 1 et une source auxiliaire de stimulation.

Après enlèvement de la tige 15, l'étanchéité totale du dispositif est restaurée.

La surface inclinée 13a, par un effet de coin, contribue à un meilleur contact entre le manchon 13 et la tige 15.

Il est à noter que la bague 14 ou analogue pourrait être supprimée, ladite structure traversable de manière étanche en vue de l'accès au manchon 13 étant alors formée par les deux tronçons 2a,2b en recouvrement, l'effet de constriction élastique opéré par la gaine 2b sur la gaine sous-jacente 2a assurant l'étanchéité désirée tout en permettant l'insertion de la tige 15 pour contacter le manchon 13.

La figure 5 illustre encore un autre mode de réalisation appliqué à une sonde unipolaire, dans lequel le conducteur électrique 23 forme un coude au niveau duquel il est en contact avec une pièce en matériau conducteur de l'électricité 24 agencée pour former une dérivation 25 dans l'axe de la partie 23a du conducteur tournée vers l'électrode de stimulation, l'autre partie 23b du conducteur, de l'autre côté du coude, étant connectée à la fiche 26 de liaison de la sonde au stimulateur cardiaque symbolisé en 27.

La pièce 24 est agencée à l'intérieur de la partie 23 du conducteur et sort de la spirale formée par le conducteur 23 pour constituer ladite dérivation 25.

La pièce 24, au niveau de la dérivation 25, est recouverte d'une structure isolante en forme de gaine 28 dont l'extrémité est munie d'une surépaisseur ou lentille L en matériau, identique ou non à celui de la gaine environnante, susceptible d'être traversé de manière étanche, par exemple par une tige 15 d'un tournevis, c'est-à-dire susceptible, après retrait de la tige, de recouvrer son entière étanchéité, le trou de passage de la tige se refermant sur lui-même du fait de l'élasticité du matériau.

La surépaisseur ou lentille L peut être remplacée par un orifice 28a obturé par un élément amovible 28b, comme illustré par la figure 6.

La pièce 24 peut être pleine et comporter à son extrémité, comme la pièce 17, une empreinte 19 de réception de l'extrémité de la tige 15.

La pièce 24 peut aussi être tubulaire comme celle représentée en 24' sur la figure 10 et conformée, à son extrémité externe, en fiche de connexion femelle pour recevoir l'extrémité de la tige 15. Une telle pièce tubulaire 24' permet d'utiliser avantageusement, comme organe de connexion électrique auxiliaire, le mandrin métallique utilisé habituellement pour mettre en place la sonde dans le coeur. Le mandrin est tout simplement glissé dans la pièce tubulaire 24', au travers de la lentille L, puis engagé sans aucune difficulté dans la partie 23a du conducteur 23, laquelle se trouve dans l'axe de la pièce 24'. Le mandrin ainsi engagé est en contact avec le conducteur 23 et peut assurer la stimulation auxiliaire de l'électrode de la sonde.

Les parties 23a et 23b du conducteur 23 peuvent former entre elles en angle d'inflexion variable.

Tous les modes de réalisation représentés et décrits ci-dessus permettent d'assurer une liaison électrique momentanée entre le conducteur central de la sonde et une source externe, dans des conditions de fiabilité et d'étanchéité pour ce qui concerne la réutilisation de la sonde, donnant toute satisfaction.

Bien entendu, l'invention n'est pas limitée à ces modes de réalisation mais en couvre au contraire toutes les variantes notamment en ce qui concerne les nature, formes et dimensions des manchons 5,6, 13, du plot de connexion 17, de la pièce de dérivation 24,24', et des éléments pouvant être traversés de manière étanche (L,11,14) susceptibles d'être interposés entre la tige métallique indépendante (15) de liaison et lesdits éléments en matériau conducteur de l'électricité (5,13) ou pièce (24,24'), agencés à l'intérieur du cordon et au contact permanent avec le conducteur interne de la sonde.

En particulier, le principe de la dérivation illustré par la figure 5 peut s'appliquer à des sondes bi-polaires.

La figure 6 illustre une application à une sonde à deux conducteurs électriques coaxiaux concentriques, à savoir un conducteur externe 29 relié à l'électrode proximale (non représentée), à la manière connue, et un conducteur interne 30 relié à l'électrode distale (non représentée).

L'ensemble des deux conducteurs 29,30 forme un coude 31. A hauteur de ce coude est agencée une pièce tubulaire 24' en matériau conducteur de l'électricité, engagée à l'intérieur du conducteur interne 30 dans le prolongement de la partie 30a de ce conducteur tournée vers l'électrode distale.

La pièce 24' traverse le conducteur externe 29 en étant isolée électriquement de ce dernier et constitue une dérivation 25 similaire à celle de la figure 5, avec une gaine isolante 28 et un orifice d'extrémité 28a obturé par un élément amovible 28b.

Les parties coudées 29b,30b des conducteurs sont connectées à la manière habituelle à une fiche mâle double 32 de connexion à un stimulateur cardiaque (non représenté).

Ce mode de réalisation s'utilise d'une manière analogue à celle de la figure 5 pour stimuler provisoirement le coeur via l'électrode distale, l'élément 28b étant enlevé pour introduire la tige 15 ou le mandrin.

L'orifice 28a et l'élément d'obturation 28b peuvent être remplacés par la surépaisseur ou lentille traversable L de la figure 5.

La figure 7 illustre une application à une sonde bipolaire à deux spirales de même diamètre, imbriquées.

L'un des conducteurs (33) est isolé et relié à l'électrode de stimulation proximale 34, cependant que l'autre conducteur (35) n'est pas isolé et est relié à l'électrode de stimulation distale 36.

Les deux conducteurs 33,35 forment un coude 31 à hauteur duquel une pièce en matériau conducteur de l'électricité tubulaire 24' traverse les deux spirales en étant engagée à l'intérieur de celles-ci dans le prolongement des parties de spirales reliées aux électrodes 34,36 et forme une dérivation 25 similaire à celle des figures 5,6, avec un revêtement isolant (non représenté) et une surépaisseur ou lentille L (non représentée) d'accès à la pièce tubulaire 24', par l'intermédiaire d'une tige 15 de tourne-vis ou dudit mandrin métallique.

Les parties coudées (33a, 35a) des conducteurs spiralés sont reliées à la manière habituelle à une fiche mâle double 32 de connexion à un stimulateur cardiaque (non représenté).

Le mode d'utilisation du dispositif de la figure 7 est identique à celui des dispositifs des figures 5 et 6.

La pièce tubulaire 24' des dispositifs des figures 6 et 7 peut bien entendu être remplacée par la pièce pleine 24 de la figure 5.

La pièce 24' peut également être connectée au conducteur relié à l'électrode de stimulation proximale en respectant les isolations nécessaires entre la pièce et l'autre conducteur.

Les pièces 24 et 24' peuvent enfin être connectées sur l'un des deux conducteurs coudés d'une sonde à deux conducteurs spiralés parallèles.

Le pôle auxiliaire de connexion des sondes selon l'invention peut-être utilisé dans toute autre circonstance de manipulation du boîtier (par exemple réenfouissement), qu'il y ait déconnection ou changement ou non dudit boîtier.

Dans l'application aux sondes de défibrillateurs implantables, munies de trois conducteurs côte à côte ou concentriques, le pôle auxiliaire de connexion est de préférence relié au conducteur de stimulation de l'électrode distale. Il permet également de vérifier le fonctionnement du défibrillateur.

Selon les applications, le pôle auxiliaire de connexion permet aussi bien d'appliquer des stimuli ou un potentiel électrique au conducteur ou à l'un des conducteurs de toute sonde, cardiaque ou non, ou analogue, en substitution des stimulis ou potentiels normaux ou en superposition à ceux-ci, que de receuillir tout signal électrique en provenance du boîtier ou de la ou des électrodes.

## Revendications

1. Sonde de stimulation, notamment cardiaque, à pôle auxiliaire de connexion électrique, du type comprenant un cordon souple constitué d'au moins un conducteur électrique spiralé (23) et entouré d'une gaine (2) en matériau isolant et relié, à une extrémité de la sonde, à une tête amovible de connexion au boîtier du stimulateur cardiaque et, à l'autre extrémité, à un dispositif à électrode de stimulation, ladite sonde comportant une structure d'accès étanche audit conducteur électrique (23) de manière à permettre à un moyen (15) de connexion électrique auxiliaire d'assurer la liaison électrique entre ledit conducteur (23) et une source externe de stimulation, caractérisée en ce que ledit conducteur électrique (23) forme un coude au niveau duquel est agencée ladite structure d'accès étanche, celle-ci étant constituée par une pièce (24, 24') en matériau conducteur de l'électricité, disposée en contact direct avec ledit conducteur (23) en formant une dérivation (25) dans l'axe de la partie dudit conducteur (23) tournée vers l'électrode de stimulation, recouverte d'une structure de recouvrement étanche susceptible, à l'extrémité libre de ladite pièce (24, 24'), d'être traversée par ledit moyen (15) de connexion électrique auxiliaire, ce dernier venant alors en contact électrique avec ladite pièce (24, 24').

2. Sonde de stimulation selon la revendication 1, caractérisée en ce que ladite structure de recouvrement est constituée par un prolongement (28) de la gaine du cordon, muni d'un orifice (28a) d'accès à ladite pièce en matériau conducteur de l'électricité (24, 24') de la dérivation (25), disposé dans l'axe de cette dernière et pourvu d'un élément amovible d'obturation (28b)

3. Sonde de stimulation selon la revendication 1, caractérisée en ce que ladite structure de recouvrement est constituée par un prolongement (28) de la gaine du cordon, muni à son extrémité d'une surépaisseur ou inclusion rapportée (L) en matériau traversable de manière étanche par ledit moyen (15).

4. Sonde de stimulation selon la revendication 3, caractérisée en ce que la surépaisseur ou inclusion rapportée (L) est munie d'un passage préformé étanche pouvant être traversé par ledit moyen (15).

5. Sonde de stimulation selon l'une des revendications 2 à 4, caractérisée en ce que la pièce en matériau conducteur de l'électricité (24) est munie à son extrémité libre d'une empreinte (19) de réception de l'extrémité d'une tige métallique indépendante formant ledit moyen (15).

6. Sonde de stimulation selon l'une des revendications 2 à 4, caractérisée en ce que la pièce en matériau conducteur de l'électricité (24') est tubulaire et permet la libre réception d'un mandrin de mise en place de la sonde dans le coeur.

7. Sonde de stimulation selon l'une des revendications 2 à 6, comportant au moins deux conducteurs spiralés coaxiaux (29, 30), caractérisée en ce que ladite pièce (24, 24') en matériau conducteur de l'électricité est connecté à l'un des conducteurs (29, 30).

8. Sonde de stimulation selon l'une des revendications 2 à 6, comportant au moins deux conducteurs spiralés parallèles (33, 35), caractérisée en ce que ladite pièce (24, 24') en matériau conducteur de l'électricité est connectée à l'un des conducteurs spiralés (33, 35), de préférence celui relié à l'électrode de stimulation distale.

9. Sonde de stimulation, notamment cardiaque, à pôle auxiliaire de connexion électrique, du type comprenant un cordon souple constitué d'au moins un conducteur électrique spiralé (1) et entouré d'une gaine (2) en matériau isolant et relié, à une extrémité de la sonde, à une tête amovible de connexion au boîtier du stimulateur cardiaque et, à l'autre extrémité, à un dispositif à électrode de stimulation, ladite sonde comportant une structure d'accès étanche audit conducteur électrique (1) de manière à permettre à un moyen (15) de connexion électrique auxiliaire d'assurer la liaison électrique entre ledit conducteur (1) et une source externe de stimulation, caractérisée en ce que ledit conducteur électrique (1) comporte, au droit de la structure d'accès étanche, un manchon (5) en matériau conducteur de l'électricité entourant ledit conducteur, ledit manchon (5) étant recouvert par la gaine (2) avec interposition d'une pièce (6) en matériau rigide non conducteur de l'électricité, ladite structure d'accès étant constituée par une vis (7) en matériau conducteur de l'électricité dont la tête est recouverte d'une pastille en matériau isolant, ladite vis étant engagée dans un puits ménagé dans la gaine (2) et dans ladite pièce (6), de manière à venir par vissage en contact avec ledit manchon (5), sans rupture de l'étanchéité entre l'intérieur et l'extérieur de la gaine, ladite pastille isolante étant traversable de manière étanche par ledit moyen (15), permettant de la sorte à ce dernier de venir en contact électrique avec le conducteur, via la vis (7) et le manchon (5).

10. Sonde de stimulation, notamment cardiaque, à pôle auxiliaire de connexion électrique, du type comprenant un cordon souple constitué d'au moins un conducteur électrique spiralé (1) et entouré d'une gaine (2) en matériau isolant et relié, à une extrémité de la sonde, à une tête amovible de connexion au boîtier du stimulateur cardiaque et, à l'autre extrémité, à un dispositif à électrode de stimulation, ladite sonde comportant une structure d'accès étanche audit conducteur électrique (1) de manière à permettre à un moyen (15) de connexion électrique auxiliaire d'assurer la liaison électrique entre ledit conducteur (1) et une source externe de stimulation, caractérisée en ce que ledit conducteur électrique (1) comporte, au droit de la structure d'accès étanche, un manchon (13) en matériau conducteur de l'électricité entourant ledit conducteur, ladite gaine (2) étant séparée en deux tronçons (2a, 2b), le premier (2a) venant en butée contre ledit manchon (13) et le second (2b) recouvrant ledit manchon (13) ainsi que l'extrémité du premier tronçon (2a), ledit second tronçon (2b) assurant l'étanchéité par constriction, ledit moyen (15) étant susceptible d'être inséré sous le second tronçon (2b) jusqu'à venir en contact avec le manchon (5).

## Claims

1. Stimulation probe, notably for cardiac stimulation, with an auxiliary electrical connection pole, of the type comprising a flexible lead consisting of at least one spirally wound electrical conductor (23) surrounded by a sheath (2) made of insulating material and connected, at one end of the probe, to a removable head for connection to the casing of the cardiac stimulator and, at the other end, to a device with a stimulation electrode, the probe having a structure for sealed access to the electrical conductor (23) so as to enable an auxiliary electrical connection means (15) to provide the electrical connection between the conductor (23) and an external stimulation source, characterised in that the electrical conductor (23) forms a bend, at which is arranged the sealed access structure, the latter consisting of a piece (24,24') made of electrically conductive material, disposed in direct contact with the said conductor (23) whilst forming a stub (25) in line with the part of the conductor (23) turned towards the stimulation electrode, covered with a sealed covering structure able, at the free end of the said piece (24,24'), to have the auxiliary electrical connection means (15) pass through it, the latter then coming into electrical contact with the said piece (24,24').

2. Stimulation probe according to Claim 1, characterised in that the covering structure consists of an extension (28) of the sheath of the lead, provided with an orifice (28a) for access to the piece made of electrically conductive material (24,24') of the stub (25), disposed in line with the latter and provided with a removable closure element (28b).

3. Stimulation probe according to Claim 1, characterised in that the covering structure consists of an extension (28) of the sheath of the lead, provided at its end with a thicker part or attached inclusion (L) made of material which the means (15) can pass through sealingly.

4. Stimulation probe according to Claim 3, characterised in that the thicker part or attached inclusion (L) is provided with a sealed preformed passage which the means (15) can pass through.

5. Stimulation probe according to one of Claims 2 to 4, characterised in that the piece made of electrically conductive material (24) is provided at its free end with an indentation (19) for receiving the end of an independent metallic rod forming said means (15).

6. Stimulation probe according to one of Claims 2 to 4, characterised in that the piece made of electrically conductive material (24') is tubular and allows the free reception of a mandrin for fitting the probe in the heart.

7. Stimulation probe according to one of Claims 2 to 6, having at least two coaxial spirally wound conductors (29, 30), characterised in that the said piece (24, 24') made of electrically conductive material is connected to one of the conductors (29, 30).

8. Stimulation probe according to one of Claims 2 to 6, having at least two parallel spirally wound conductors (33, 35), characterised in that the said piece (24, 24') made of electrically conductive material is connected to one of the spirally wound conductors (33, 35), preferably the one connected to the distal stimulation electrode.

9. Stimulation probe, notably for cardiac stimulation, with an auxiliary electrical connection pole, of the type comprising a flexible lead consisting of at least one spirally wound electrical conductor (1) surrounded by a sheath (2) made of insulating material and connected, at one end of the probe, to a removable head for connection to the casing of the cardiac stimulator and, at the other end, to a device with a stimulation electrode, the said probe having a structure for sealed access to the said electrical conductor (1) so as to enable an auxiliary electrical connection means (15) to provide the electrical connection between the said conductor (1) and an external stimulation source, characterised in that the said electrical conductor (1) has, in line with the sealed access structure, a sleeve (5) made of electrically conductive material surrounding the said conductor, the said sleeve (5) being covered by the sheath (2) with a piece (6) made of electrically non-conductive rigid material being interposed, the said access structure consisting of a screw (7) made of electrically conductive material, whose head is covered with a pellet made of insulating material, the said screw being engaged in a well formed in the sheath (2) and in the said piece (6), so as to come, through screwing, into contact with the said sleeve (5), without breaking the seal between the inside and outside of the sheath, the said means (15) being able to pass sealingly through the said insulating pellet, in this way enabling the said means to come into electrical contact with the conductor, via the screw (7) and sleeve (5).

10. Stimulation probe, notably for cardiac stimulation, with an auxiliary electrical connection pole, of the type comprising a flexible lead consisting of at least one spirally wound electrical conductor (1) surrounded by a sheath (2) made of insulating material and connected, at one end of the probe, to a removable head for connection to the casing of the cardiac stimulator and, at the other end, to a device with a stimulation electrode, the said probe having a structure for sealed access to the said electrical conductor (1) so as to enable an auxiliary electrical connection means (15) to provide the electrical connection between the said conductor (1) and an external stimulation source, characterised in that the said electrical conductor (1) has, in line with the sealed access structure, a sleeve (13) made of electrically conductive material surrounding the said conductor, the said sheath (2) being separated into two sections (2a, 2b), the first (2a) coming into abutment against the said sleeve (13) and the second (2b) covering the said sleeve (13) and the end of the first section (2a), the said second section (2b) providing the seal by constriction, the said means (15) being able to be inserted underneath the second section (2b) until it comes into contact with the sleeve (13).

## Patentansprüche

1. Stimulationssonde, insbesondere für einen Herzschrittmacher, mit einem zusätzlichen Pol zur elektrischen Verbindung, eines Typs umfassend eine biegsame Leitung, die aus wenigstens einem spiralförmigen elektrischen Leiter (23) besteht, der mit einer Ummantelung (2) aus elektrisch isolierendem Material umgeben und an einem Ende der Sonde mit einem lösbaren Kopf zum Verbinden mit dem Gehäuse des Herzschrittmachers und am anderen Ende mit einer Elektrodenstimulationsvorrichtung verbunden ist, wobei die Sonde eine dichte Anschlußstruktur zum elektrischen Leiter (23) derart umfaßt, daß es einem Mittel (15) zur zusätzlichen elektrischen Verbindung ermöglicht wird, die elektrische Verbindung zwischen dem Leiter (23) und einer äußeren Stimulationsquelle sicherzustellen, dadurch gekennzeichnet, daß der elektrische Leiter (23) einen Winkel bildet, in dessen Bereich die dichte Anschlußstruktur angeordnet ist, die durch ein Teil (24, 24') aus elektrisch leitendem Material gebildet wird, in direktem Kontakt mit dem Leiter (23) unter Ausbildung einer Abzweigung (25) in der Achse des Teils des Leiters (23) steht, der zur Stimulationselektrode gerichtet ist, und von einer dichten Ummantelungsstruktur bedeckt ist, die in der Lage ist, am freien Ende des Teils (24, 24') durch das Mittel (15) zur zusätzlichen elektrischen Verbindung durchdrungen zu werden, wodurch letzteres in elektrischen Kontakt mit dem Teil (24, 24') gelangt.

2. Stimulationssonde nach Anspruch 1, dadurch gekennzeichnet, daß die Ummantelungsstruktur durch eine Verlängerung (28) der Ummantelung der Leitung gebildet wird, die mit einer Zutrittsöffnung (28a) zu dem Teil (24, 24') aus elektrisch leitendem Material des Winkels (25) versehen ist, die in der Achse des letzteren angeordnet und mit einem lösbaren Verschlußelement (28b) versehen ist.

3. Stimulationssonde nach Anspruch 1, dadurch gekennzeichnet, daß die Ummantelungsstruktur durch eine Verlängerung (28) der Ummantelung der Leitung gebildet wird, die an ihrem Ende mit einer Überdicke oder einem eingesetzten Einschluß (L) aus durch das Mittel (15) in dichtender Weise durchdringbarem Material versehen ist.

4. Stimulationssonde nach Anspruch 3, dadurch gekennzeichnet, daß die Überdicke oder der eingesetzte Einschluß (L) mit einem vorgeformten dichten Durchtritt versehen ist, der von dem Mittel (15) durchdringbar ist.

5. Stimulationssonde nach einem der Ansprüche 2 bis 4, dadurch gekennzeichnet, daß das Teil (24) aus elektrisch leitendem Material an seinem freien Ende mit einer Vertiefung (19) zur Aufnahme des Endes eines das Mittel (15) bildenden, unabhängigen, metallischen Schafts versehen ist.

6. Stimulationssonde nach einem der Ansprüche 2 bis 4, dadurch gekennzeichnet, daß das Teil (24') aus elektrisch leitendem Material rohrförmig ist und die freie Aufnahme eines Dorns zum Einsetzen der Sonde in das Herz ermöglicht.

7. Stimulationssonde nach einem der Ansprüche 2 bis 6, umfassend wenigstens zwei koaxiale spiralförmige Leiter (29, 30), dadurch gekennzeichnet, daß das Teil (24, 24') aus elektrisch leitendem Material mit einem der Leiter (29, 30) verbunden ist.

8. Stimulationssonde nach einem der Ansprüche 2 bis 6, umfassend wenigstens zwei parallele spiralförmige Leiter (33, 35), dadurch gekennzeichnet, daß das Teil (24, 24') aus elektrisch leitendem Material mit einem der spiralförmigen Leiter (33, 35) verbunden ist, und zwar vorzugsweise mit demjenigen, der mit der distalen Stimulationselektrode verbunden ist.

9. Stimulationssonde, insbesondere für einen Herzschrittmacher, mit einem zusätzlichen Pol zur elektrischen Verbindung, eines Typs umfassend eine biegsame Leitung, die aus wenigstens einem spiralförmigen elektrischen Leiter (1) besteht, der mit einer Ummantelung (2) aus elektrisch isolierendem Material umgeben und an einem Ende der Sonde mit einem lösbaren Kopf zum Verbinden mit dem Gehäuse des Herzschrittmachers und am anderen Ende mit einer Elektrodenstimulationsvorrichtung verbunden ist, wobei die Sonde eine dichte Anschlußstruktur zum elektrischen Leiter (1) derart umfaßt, daß es einem Mittel (15) zur zusätzlichen elektrischen Verbindung ermöglicht wird, die elektrische Verbindung zwischen dem Leiter (1) und einer äußeren Stimulationsquelle sicherzustellen, dadurch gekennzeichnet, daß der elektrische Leiter (1) an der dichten Anschlußstruktur eine den Leiter umgebende Hülse (5) aus elektrisch leitendem Material umfaßt, die von der Ummantelung (2) unter Zwischenschaltung eines Teils (6) aus starrem, nicht elektrisch leitenden Material ummantelt ist, wobei die Anschlußstruktur durch eine Schraube (7) aus elektrisch leitendem Material gebildet wird, deren Kopf durch ein Plättchen aus isolierendem Material bedeckt ist, wobei die Schraube in eine Bohrung, die in der Ummantelung (2) und in dem Teil (6) angebracht ist, derart eingesetzt ist, daß sie durch Schrauben mit der Hülse (5) in Kontakt gelangt, ohne die Dichtigkeit zwischen dem Inneren und dem Äußeren der Ummantelung zu zerstören, wobei das isolierende Plättchen, das abdichtend von dem Mittel (15) durchdringbar dies derart ermöglicht, daß letzteres in elektrischen Kontakt mit dem Leiter über die Schraube (7) und die Hülse (5) gelangt.

10. Stimulationssonde, insbesondere für einen Herzschrittmacher, mit einem zusätzlichen Pol zur elektrischen Verbindung, eines Typs umfassend eine biegsame Leitung, die aus wenigstens einem spiralförmigen elektrischen Leiter (1) besteht, der mit einer Ummantelung (2) aus elektrisch isolierendem Material umgeben und an einem Ende der Sonde mit einem lösbaren Kopf zum Verbinden mit dem Gehäuse des Herzschrittmachers und am anderen Ende mit einer Elektrodenstimulationsvorrichtung verbunden ist, wobei die Sonde eine dichte Anschlußstruktur zum elektrischen Leiter (1) derart umfaßt, daß es einem Mittel (15) zur zusätzlichen elektrischen Verbindung ermöglicht die elektrische Verbindung zwischen dem Leiter (1) und einer äußeren Stimulationsquelle sicherzustellen, dadurch gekennzeichnet, daß der elektrische Leiter (1) an der dichten Anschlußstruktur eine den Leiter umgebende Hülse (13) aus elektrisch leitendem Material umfaßt, wobei die Ummantelung (2) in zwei Teilabschnitte (2a, 2b) getrennt ist, von denen der erste (2a) gegen die Hülse (13) stößt und der zweite (2b) die Hülse (13) ebenso wie das Ende des ersten Teilabschnitts (2a) bedeckt, wobei der zweite Teilabschnitt (2b) die Dichtigkeit durch Einschnürung sicherstellt, wobei das Mittel (15) unter den zweiten Teilabschnitt (2b), bis es in Kontakt mit der Hülse (13) gelangt, einsetzbar ist.
